# EUROPEAN PATENT SPECIFICATION

(11) **EP 4 561 833 B1**
(45) Date of publication and mention of the grant of the patent: **27.05.2026**
(21) Application number: 23761698.2
(22) Date of filing: 28.07.2023
(51) Int. Cl.: B33Y 10/00, C08G 77/04, C08J 9/02, C08J 9/12, C08J 9/00, C08J 9/08

(54) **SILICONE FOAM COMPOSITIONS**
SILICONSCHAUMZUSAMMENSETZUNGEN
COMPOSITIONS DE MOUSSE DE SILICONE

(30) Priority: 28.07.2022 US 202263393084 P
(43) Date of publication of application: 04.06.2025
(73) Proprietor: Elkem Silicones USA Corp., East Brunswick, NJ 08816 (US)
(72) Inventor: GAINES, Kyle, Ann Arbor, Michigan 48108 (US); KIHARA, Matthew, York, SC 29745 (US); BROWN, Leeanne, Rock Hill, SC 29732 (US); BULGER, Lindsay, Fort Mill, SC 29715 (US); O'NEIL, Virginia, Pinnacle, NC 27043 (US); MALDONADO, Mario, Charlotte, NC 28273 (US)
(74) Representative: Menville, Laure
(86) International application number: PCT/US2023/071209
(87) International publication number: WO 2024/026456

(56) References cited:
- WO-A1-2020/072374
- CN-A- 104 530 712
- US-A1- 2014 024 731

## Description

### Cross-Reference to Related Applications

The instant application claims priority to U.S. Provisional Application No. 63/393,084, filed on July 28, 2022.

### Technical Field

The present invention relates to the technical field of silicone compositions. More specifically, it relates to new silicone foams composition having a reduced density.

### Background

Silicone elastomers have attracted a great interest as cured silicones have properties such as high elasticity, flexibility at low and high temperatures, high gas permeability, very low glass transition temperatures (Tg around -120°C), very good dielectric properties and fire resistance properties.

In electronic devices, by virtue of their diverse and excellent properties, silicone elastomers can be used in a wide variety of applications. Cured silicone elastomers are indeed used for potting or encapsulating, sealing, bonding or coating various kinds of components in harsh environments as well as high-end precision/sensitive electronic devices such as light-emitting diodes (LED), displays, photovoltaic junction boxes in solar cell modules, diodes, semiconductor devices, relays, sensors, automotive stabilizers, automotive electronic control units (ECUs), etc., mainly for electrical, thermal or acoustic insulation, moisture or dust protection, or shock absorption.

As silicone foams can provide significant weight savings when compared to non-foamed elastomers, in recent years considerable efforts have been invested in developing methods of introducing porosity in silicone cured materials without causing detrimental effects on their mechanical properties.

Articles made of silicone foam are already known in various fields of application, such as thermal and/or sound insulation, the production of flexible joints, use as damping elements, shock absorbing, and the like. The markets are various: construction, transportation, electronics, energy production, industrial textiles, performance apparel, domestic electrical appliance, etc.

For example, the transportation industry shows some interest in silicone foams which are of low density while retaining excellent mechanical and fire resistance properties. Articles made of silicone foams can be used as automotive parts such as hood buffering pads, engine vibration insulators, seats, protective textiles or sheeting, vibration & noise dampening pieces, etc. The patent application US 2022/0275207 discloses a silicone foam with a density of less than 0.20 g/cm³, and which exhibit good physical, mechanical and fire-resistance properties.

As another example, new energy storage means are considering the use of silicone foams because of their excellent thermal insulating properties, and their good moisture resistance with a supplementary advantage of being a lightweight alternative to traditional elastomeric encapsulants and sealants. For example, the patent application US 2018/223070 filed by Elkem Silicones USA Corp. discloses the use of silicone syntactic foam for thermally insulating a secondary battery pack and further minimizing the propagation of thermal runaway.

Besides transportation industry and energy storage field, silicones foams can be used in various other markets for the production of articles. For example, cosmetic puffs, medical liquid-absorbing materials, various filters, various sealing elements such as packing, gaskets, o-rings, etc., may be mentioned. They may be foamed articles per se, or may be composites or laminates with metals, organic resins, or elastic materials. WO 2020/072374 A1 discloses a kit for preparing a customizable flesh simulating silicone foam, wherein a chemical blowing agent is employed. Other well-known applications of foamed articles are fixing rollers, fixing belts and the like which fix toners on paper by means of heat and/or pressure in image-forming apparatuses of electrophotographic types such as copying machines, printers, facsimile machines, and the like. Additionally, silicone foams can be used in textile and furnishing field: insulation-coated textile, footwear and garments insulation, padding.

### Summary of the Invention

The present invention relates to a silicone foam obtained from a blowable crosslinkable silicone composition comprising:
- at least one organopolysiloxane **A** having at least two alkenyl groups bonded to silicon per molecule;
- at least one organosilicon compound **B** having at least two, preferably at least three, hydrogen atoms bonded to silicon per molecule;
- at least one hydrosilylation catalyst **C;**
- at least one porogenic agent **D** which is water, a hydrogel, or an aqueous silicone emulsion;
- at least one chemical blowing agent **E;** and
- at least one linear polydimethylsiloxane **F** which has a dynamic viscosity at 25°C of between 50 mPa.s and 100000 mPa.s.

The silicone foam as defined above can be described as a "dual-blowing" silicone foam:
- on one side, the porogenic agent **D,** which is water, a hydrogel, or an aqueous silicone emulsion, generates hydrogen bubbles while incorporated into the polyaddition-crosslinking silicone composition comprising the organopolysiloxane **A** carrying alkenyl groups bonded to the silicon, the organosilicon compound **B** containing hydrogen atoms bonded to the silicon, and the hydrosilylation catalyst **C,** and
- on the other side, the chemical blowing agent **E** releases gas, typically carbon dioxide, by decomposition.

The inventors discovered that the resulting dual-blowing silicone foam shows a low density, and the foam cellular structure was considerably more homogenous and stable than just using one of the blowing agents or the other, while also retaining a soft shock-resistant feel.

### Detailed Description of the Invention

All the viscosities under consideration in the present specification correspond to a dynamic viscosity magnitude that is measured, in a manner known per se, at 25°C, at a sufficiently low shear rate gradient so that the viscosity measured with a machine of Brookfield type is independent of the rate gradient.

Unless otherwise specified, the contents in % or ppm are by weight.

The blowable crosslinkable silicone composition according to the present invention comprises at least one organopolysiloxane **A** having at least two alkenyl groups bonded to silicon per molecule. Preferably, the organopolysiloxane **A** exhibits, per molecule, at least two C₂₋₆ alkenyl groups bonded to the silicon. It can consists of at least two siloxy units of following formula: YₐR¹_{b}SiO_{(4-a-b)/2}
in which:
- Y is a C₂₋₆ alkenyl, preferably vinyl,
- R¹ is a monovalent hydrocarbon group having from 1 to 12 carbon atoms, preferably selected from the alkyl groups having from 1 to 8 carbon atoms, such as the methyl, ethyl or propyl groups, cycloalkyl groups having from 3 to 8 carbon atoms and aryl groups having from 6 to 12 carbon atoms,
- a = 1 or 2, b = 0, 1 or 2 and the sum a + b = 2 or 3,

and optionally units of following formula: R¹_{c}SiO_{(4-c)/2}
in which R¹ has the same meaning as above and c = 0, 1, 2 or 3.

Preferably, the organopolysiloxane **A** can have a dynamic viscosity at 25°C of between 100 mPa.s and 120,000 mPa.s, preferably between 100 mPa.s and 80,000 mPa.s, more preferentially between 1,000 mPa.s and 50,000 mPa.s, and even more preferably between 5,000 mPa.s and 20,000 mPa.s. Said organopolysiloxane **A** can preferably be referred to as an organopolysiloxane oil.

The organopolysiloxane **A** can be a linear organopolysiloxane, a cyclic organopolysiloxane or a branched organopolysiloxane (resin). The blowable crosslinkable silicone composition according to the present invention can comprise a mixture of different organopolysiloxanes **A.**

According to one embodiment, the organopolysiloxane **A** can be a linear organopolysiloxane. Linear organopolysiloxanes exhibit a linear structure essentially formed of D or D^{Vi} siloxyl units, and of terminal M or M^{Vi} siloxyl units, with D, D^{Vi}, M and M^{Vi} defined as follows: D: R¹₂SiO_{2/2} siloxyl unit, D^{Vi}: siloxyl unit selected from the group consisting of Y₂SiO_{2/2} or YR¹SiO_{2/2} siloxyl units, M: R¹₃SiO_{1/2} siloxyl unit, M^{Vi}: siloxyl unit selected from the group consisting of the YR¹₂SiO_{1/2} and Y₂R¹SiO_{1/2}; the symbols Y and R¹ are as described above.

As examples of terminal "M or M^{Vi}" units, mention may be made of the trimethylsiloxy, dimethylphenylsiloxy, dimethylvinylsiloxy or dimethylhexenylsiloxy groups.

As examples of "D or D^{V}" units, mention may be made of the dimethylsiloxy, methylphenylsiloxy, methylvinyl-siloxy, methylbutenylsiloxy, methylhexenylsiloxy, methyldecenylsiloxy or methyldecadienylsiloxy groups.

Examples of linear or cyclic organopolysiloxanes which can be organopolysiloxane **A** according to the invention are:
- a poly(dimethylsiloxane) comprising dimethylvinyl-silyl terminations;
- a poly(dimethylsiloxane-co-methylphenylsiloxane) comprising dimethylvinylsilyl terminations;
- a poly(dimethylsiloxane-co-methylvinylsiloxane) comprising dimethylvinylsilyl terminations;
- a poly(dimethylsiloxane-co-methylvinylsiloxane) comprising trimethylsilyl terminations; and
- a cyclic poly(methylvinylsiloxane).

Preferably, the organopolysiloxane **A** has a content by weight of alkenyl unit of between 0.001% and 30%, preferably between 0.01% and 10%, preferably between 0.02% and 5%.

According to a preferred embodiment, the organopolysiloxane **A** contains terminal dimethylvinylsilyl units, and even more preferably the organopolysiloxane **A** is a poly(dimethylsiloxane) comprising terminal dimethylvinylsilyl groups. The number of dimethylsiloxane units can be comprised between 5 to 1000, and preferably from 100 to 600.

According to another embodiment, the organopolysiloxane **A** can be a branched organopolysiloxane (i.e. a resin) comprising C₂₋₆ alkenyl units. It is preferably selected from the group consisting of the silicone resins of following formulas:
- M^{Vi}Q, where the alkenyl groups bonded to silicon atoms are carried by the M groups,
- MM^{Vi}Q, where the alkenyl groups bonded to silicon atoms are carried by a part of the M units,
- MD^{Vi}Q, where the alkenyl groups bonded to silicon atoms are carried by the D groups,
- MDD^{Vi}Q, where the alkenyl groups bonded to silicon atoms are carried by a part of the D groups,
- MM^{Vi}TQ, where the alkenyl groups bonded to silicon atoms are carried by a part of the M units,
- MM^{Vi}DD^{Vi}Q, where the hydrogen atoms bonded to silicon atoms are carried by a part of the M and D units,
- and their mixtures,
with M, M^{Vi}, D and D^{Vi} as defined above, T: siloxyl unit of formula R¹SiO_{3/2}, and Q: siloxyl unit of formula SiO_{4/2}, where R¹ has the same meaning as above.

According to a preferred embodiment, the blowable crosslinkable silicone composition according to the present invention comprises a mixture of at least one linear organopolysiloxanes as defined above and of at least one branched organopolysiloxane (i.e. resin) as defined above. For example, the blowable crosslinkable silicone composition according to the present invention can comprise a mixture of a linear poly(dimethylsiloxane) comprising dimethylvinyl-silyl terminations and of a silicone resins of formula M^{Vi}Q, MM^{Vi}Q, MD^{Vi}Q, MDD^{Vi}Q, MM^{Vi}TQ, or MM^{Vi}DD^{Vi}Q, preferably M^{Vi}Q, MM^{Vi}Q, MD^{Vi}Q, or MDD^{Vi}Q. The amount of the linear poly(dimethylsiloxane) in the blowable crosslinkable silicone composition according to the present invention can be in the range from 0.8% to 94% by weight, preferably 2.5% to 45% by weight, more preferably 3.5% to 25% by weight, of the total composition. The amount of the silicone resin in the blowable crosslinkable silicone composition according to the present invention can be in the range from 0% to 10% by weight, preferably 0.01% to 5% by weight, more preferably 0.05% to 2% by weight, of the total composition.

The blowable crosslinkable silicone composition according to the present invention further comprises at least one organosilicon compound **B** having at least two and preferably at least three hydrogen atoms bonded to silicon per molecule. The organosilicon compound **B** is preferably an organohydrogenpolysiloxane compound comprising, per molecule, at least two and preferably at least three hydrosilyl functional groups (or Si-H units).

The organosilicon compound **B** can advantageously be an organopolysiloxane comprising at least two, preferably at least three, siloxyl units of following formula: H_{d}R²ₑSiO_{(4-d-e)/2}
in which:
- the R² radicals, which are identical or different, represent a monovalent radical having from 1 to 12 carbon atoms,
- d = 1 or 2, e = 0, 1 or 2 and d + e = 1, 2 or 3;

and optionally other units of following formula: R²_{f}SiO_{(4-f)/2}
in which R² has the same meaning as above and f = 0, 1, 2 or 3.

It is understood that, in the formulas above, if several R² groups are present, they can be identical to or different from one another. Preferentially, R² can represent a monovalent radical selected from the group consisting of alkyl groups having from 1 to 8 carbon atoms, optionally substituted by at least one halogen atom, such as chlorine or fluorine, cycloalkyl groups having from 3 to 8 carbon atoms and aryl groups having from 6 to 12 carbon atoms. R² can advantageously be selected from the group consisting of methyl, ethyl, propyl, 3,3,3-trifluoropropyl, xylyl, tolyl and phenyl, and most preferentially R² is methyl.

The symbol d is preferentially equal to 1.

The organosilicon compound **B** can exhibit a linear, branched or cyclic structure. The degree of polymerization is preferably greater than or equal to 2. Generally, it is less than 5000.

When linear polymers are concerned, the latter are essentially formed of siloxyl units selected from the units of following formulas D: R²₂SiO_{2/2} or D': R²HSiO_{2/2} and of terminal siloxyl units selected from the units of following formulas M: R²₃SiO_{1/2} or M': R²₂HSiO_{1/2} where R² has the same meaning as above.

Preferably, the viscosity of the organosilicon compound **B** is between 1 mPa.s and 5,000 mPa.s, more preferentially between 1 mPa.s and 2,000 mPa.s and more preferentially still between 5 mPa.s and 1,000 mPa.s.

Examples of organohydrogenpolysiloxanes which can be organosilicon compound **B** according to the invention comprising at least two hydrogen atoms bonded to a silicon atom are:
- a poly(dimethylsiloxane) comprising hydrodimethyl-silyl terminations;
- a poly(dimethylsiloxane-co-methylhydrosiloxane) comprising trimethylsilyl terminations;
- a poly(dimethylsiloxane-co-methylhydrosiloxane) comprising hydrodimethylsilyl terminations;
- a poly(methylhydrosiloxane) comprising trimethyl-silyl terminations; and
- a cyclic poly(methylhydrosiloxane).

When the organosilicon compound **B** exhibits a branched structure, it is preferably selected from the group consisting of the silicone resins of following formulas:
- M'Q, where the hydrogen atoms bonded to silicon atoms are carried by the M groups,
- MM'Q, where the hydrogen atoms bonded to silicon atoms are carried by a part of the M units,
- MD'Q, where the hydrogen atoms bonded to silicon atoms are carried by the D groups,
- MDD'Q, where the hydrogen atoms bonded to silicon atoms are carried by a part of the D groups,
- MM'TQ, where the hydrogen atoms bonded to silicon atoms are carried by a part of the M units,
- MM'DD'Q, where the hydrogen atoms bonded to silicon atoms are carried by a part of the M and D units,
- and their mixtures,
with M, M', D and D' as defined above, T: siloxyl unit of formula R²SiO_{3/2} and Q: siloxyl unit of formula SiO_{4/2}, where R² has the same meaning as above.

Preferably, the organosilicon compound **B** has a content by weight of hydrosilyl Si-H functional groups of between 0.2% and 91%, more preferentially between 3% and 80% and more preferentially still between 15% and 70%.

Advantageously, the molar ratio of the hydrosilyl SiH functional groups of the organosilicon compound **B** to the alkene functional groups of the compound A is between 1 and 50, preferably between 2 and 30, more preferentially between 3 and 20.

According to one preferred embodiment, the blowable crosslinkable silicone composition according to the present invention comprises a mixture of at least one organosilicon compound **B1** having at least three hydrogen atoms bonded to silicon per molecule and at least one organosilicon compound **B2** having two hydrogen atoms bonded to silicon per molecule. Said organosilicon compound **B2** contains preferably terminal dimethylhydrogensilyl units, and even more preferably the organosilicon compound **B2** is a poly(dimethylsiloxane) comprising terminal dimethylhydrogensilyl groups. The number of dimethylsiloxane units within the organosilicon compound **B2** can be comprised between 1 to 200, preferably between 1 and 150, and more preferably between 3 and 120. Such organosilicon compound **B2** can be described as "chain extender" since it has the presumed effect of increasing the mesh size of the network when it is crosslinked. Besides, such organosilicon compound **B1** having three hydrogen atoms bonded to silicon per molecule or more can be described as "crosslinker". Preferably the organosilicon compound **B1** is a poly(dimethylsiloxane-co-methylhydrosiloxane) comprising trimethylsilyl terminations and/or hydrodimethylsilyl terminations.

The hydrosilylation catalyst **C** can in particular be selected from platinum and rhodium compounds but also from silicon compounds, such as those described in the patent applications WO 2015/004396 and WO 2015/004397, germanium compounds, such as those described in the patent application WO 2016/075414, or nickel, cobalt or iron complexes, such as those described in the patent applications WO 2016/071651, WO 2016/071652 and WO 2016/071654. The catalyst C is preferably a compound derived from at least one metal belonging to the platinum group. These catalysts are well known. It is possible in particular to use complexes of platinum and of an organic product described in the patents US 3,159,601, US 3,159,602 and US 3,220,972 and the European patents EP 0057459, EP 0188978 and EP 0190530, or the complexes of platinum and of vinylated organosiloxanes described in the patents US 3,419,593, US 3,715,334, US 3,377,432 and US 3,814,730.

Preferentially, the catalyst **C** is a compound derived from platinum. Preferentially, the catalyst C is a Karstedt platinum catalyst.

The blowable crosslinkable silicone composition according to the present invention comprises water, a hydrogel, or an aqueous silicone emulsion as porogenic agent **D.** The water can be added directly to the blowable crosslinkable silicone composition. Advantageously, the water can be introduced in the form of an aqueous silicone emulsion, for example a direct oil-in-water silicone emulsion or an inverse water-in-oil silicone emulsion comprising a continuous silicone oily phase, an aqueous phase and a stabilizer.

According to one embodiment, the water is introduced via an emulsion of silicone oil in water with a water content of the order of 60% by weight. When the water is introduced into the blowable crosslinkable silicone composition via an emulsion, the dispersion of the water in the blowable crosslinkable silicone composition and its stability on storage are improved.

According to one embodiment, an emulsifier can be added with the water or with the aqueous silicone emulsion. Said emulsifier can be selected by the person skilled in the art among the typical emulsifiers. It can be an anionic, cationic, amphoteric, or a nonionic emulsifier. Among these, most preferable are nonionic surfactants because they might have minimal influence on the hydrosilylation reaction. The emulsifier can be added in an amount such that the weight ratio of emulsifier vs water can be between 1:5 and 5:1, preferably between 2:1 and 1:2.

A part of the hydrosilyl functional groups of the organosilicon compound **B** will react with the water provided by the porogenic agent **D** and form the gaseous hydrogen making possible the good foaming of the composition.

The blowable crosslinkable silicone composition according to the present invention comprises at least one chemical blowing agent **E.** Preferably said chemical blowing agent **E** is at least one hydrogencarbonate salt (also commonly called "bicarbonate salt"). More preferably said chemical blowing agent **E** is selected from the group consisting of ammonium hydrogencarbonate (NH₄)HCO₃, sodium hydrogencarbonate NaHCO₃, calcium hydrogencarbonate Ca(HCO₃)₂, and mixtures thereof. Even more preferably said chemical blowing agent **E** is ammonium hydrogencarbonate.

Said chemical blowing agent **E** can have particles having a median particle size (D50) of ≤ 50 µm, and even more preferably ≤ 10 µm. According to a preferred embodiment, the particles of chemical blowing agent **E** can be grinded and sieved before use.

For the ease of application and production, the chemical blowing agent **E** can be pre-dispersed in said organopolysiloxane **A,** for example at a level from 30% to 60% by weight, with an eventual incorporation of any additive that could help to stabilize the shelf-life of the resulting composition.

The blowable crosslinkable silicone composition according to the present invention comprises at least one linear polydimethylsiloxane **F** which has a dynamic viscosity at 25°C of between 50 mPa.s and 100,000 mPa.s, preferably of between 50 mPa.s to 70,000 mPa.s, more preferably of between 100 mPa.s to 20,000 mPa.s, more preferably of between 200 mPa.s to 5,000 mPa.s, even more preferably of between 1,000 mPa.s to 2,000 mPa.s.

According to a first embodiment of the invention said linear polydimethylsiloxane **F** has the following formula:

(CH₃)₃SiO (SiO(CH₃)₂)ₙ Si(CH₃)₃ (I)

in which n is an integer from 50 to 900, and preferably from 50 to 700.

According to a second embodiment of the invention said linear polydimethylsiloxane **F** has the following formula:

(CH₃)₃SiO (SiO(CH₃)₂)ₙ Si(CH₃)₂(Y) (II)

in which Y is a C₂₋₆ alkenyl, preferably vinyl, and n is an integer from 50 to 900, and preferably from 50 to 700. Said compound has exactly one alkenyl group bonded to silicon per molecule.

Preferably, the linear polydimethylsiloxane **F** according to the present invention consists in a mixture of the linear polydimethylsiloxanes (I) and (II) as defined above. The weight ratio of (I):(II) can be comprised between 100:0 and 0:100, or between 90:10 and 10:90, or between 80:20 and 20:80, or between 70:30 and 30:70. The blowable crosslinkable silicone composition according to the present invention can be free, or substantially free of linear polydimethylsiloxane (II). The linear polydimethylsiloxane **F** according to the present invention consists in one or several linear polydimethylsiloxanes (I) as defined above.

The blowable crosslinkable silicone composition according to the present invention can optionally comprise additives. Examples of suitable additives includes: a resilient additive, a reinforcement filler, a thermally or electrically conductive filler, nanoparticles, a silicone resin, a pigment, an antimicrobial agent, a UV stabilizer, a dye, a pigment, a fragrance, a flavor, an essential oil, a flame resistant additive, a thermal stabilizer, a rheology modifier, a viscosity modifier, a thickener, an adhesion promoter, a biocide, a preservative, an enzyme, a peptide, a surface-active agent, a reactive diluent, an active pharmaceutical ingredient, an excipient or a cosmetic ingredient. The content of an additive is typically below 5 wt.%, relative to the total weight of the blowable crosslinkable silicone composition, preferably below 2.5 wt.%, more preferably below 1 wt.%. The suitable additive(s) can be selected by the person skilled in the art according to the intended application and the general knowledge of the technical field.

According to one embodiment, the blowable crosslinkable silicone composition according to the present invention can optionally comprise at least one filler, preferably a reinforcing filler, which can typically improve the mechanical strength of the cured silicone elastomer article. The filler can be precipitated silica, fumed (or pyrogenic) silicas, colloidal silicas and mixtures thereof. The specific surface area of these actively reinforcing fillers ought to be at least 10 m²/g, and preferably in the range from 50 m²/g to 400 m²/g, as determined by the BET method. In a preferred embodiment, the silica reinforcing filler is fumed silica with a specific surface area of at least 10 m²/g, and preferably in the range from 50 m²/g to 400 m²/g, as determined by the BET method. Fumed silica may be used as is, in an untreated form, but is preferably subjected to hydrophobic surface treatment. The amount of the silica reinforcing filler in the blowable crosslinkable silicone composition according to the present invention can be in the range from 0% to 10% by weight, preferably 0.01% to 5% by weight, more preferably 0.05% to 2% by weight, of the total composition.

According to one embodiment, the blowable crosslinkable silicone composition according to the present invention can optionally comprise hollow microspheres. As examples of suitable hollow microspheres, it can be cited hollow glass microspheres or hollow ceramic microspheres.

Hollow glass microspheres are sometimes termed "hollow glass beads" or "hollow glass bubbles". They are small hollow spheres of hardened silica (glass) that can vary in size and density depending on the grade. They have a shell that is thick enough to maintain structural rigidity. Due to their hollow nature, they are very lightweight, with a density that varies with size and wall thickness. In bulk they appear as a white powder. The main differences between grades are in their size, strength and density, with the strength of the microspheres being expressed in terms of their average isostatic crushing strength.

According to an embodiment, hollow glass beads are hollow borosilicate glass microspheres.

According to an embodiment, the hollow glass microspheres have a true density ranging from 0.10 g/cm³ (gram per cubic centimeter) to 0.75 g/cm³.

The terms "true density" is the quotient obtained by dividing the mass of a sample of hollow glass microspheres by the true volume of that mass of glass bubbles as measured by a gas pycnometer. The "true volume" is the aggregate total volume of the glass bubbles, not the bulk volume.

According to a preferred embodiment, hollow glass microspheres are selected from:
1. 3M^{™} Glass Bubbles Floated Series (A16/500, G18, A20/1000, H20/1000, D32/4500 and H50/10,000EPX glass bubbles products) and 3M^{™} Glass Bubbles K, S, iM and XLD Series (such as but not limited to K1, K11, K15, S15, S22, K20, K20HS, K25, S32, S32LD, S35, XLD3000, S28HS, S35, K37, S38, S38HS, S38XHS, S32HS, K46, K42HS, S42XHS, S60, S60HS, iM16K, iM30K glass bubbles products) sold by 3M Company. Said glass bubbles exhibit various crush strengths ranging from 1.72 MPa (250 psi) to 186.15 MPa (27,000 psi) at which ten percent by volume of the first plurality of glass bubbles collapses. Other glass bubbles sold by 3M such as 3M^{™} Glass Bubbles - HGS Series and 3M^{™} Glass Bubbles with Surface Treatment could also be used.
2. Hollow glass microspheres sold under the tradename SPHERICEL^{®} (products such as: 110P8, 60P18, 34P30, and 25P45) or under the tradename Q-Cel^{®} Lightweight (products such as: 6014, 6019, 7019, 6019S, 5020, 5020FPS, 7023, 7028, 2058, 6036, 7037, 7040S, 6042S, 6048 and 5070S), sold by Potters Industries Inc.

Suitable hollow glass microspheres are not surface-treated or are surface-treated. Surface-treated hollow glass microsphere can be typically hydrophobic. Surface-treatment agents can be for instance silane coupling agent such as: aminopropyltriethoxysilane, γ-glycidoxypropyltrimethoxysilane, γ-(methacryloloxy)propyltrimethoxysilane (also known as silane coupling agent KH-570) and sodium methylsiliconate.

Hollow ceramic microspheres also referred to as cenospheres are lightweight, inert, hollow sphere filled with inert air or gas, typically produced as a byproduct of coal combustion at thermal power plants. They are made largely of silica and alumina. The color of cenospheres varies from gray to almost white and their density is about 0.4 g/cm³ to 0.8 g/cm³. It flows like a liquid, with the appearance of a powder. Suitable cenospheres are not surface-treated or are surface-treated with a silane-based coupling agent such as one or more of 3-aminopropyltriethoxysilane, gamma-glycidoxypropyltrimethoxysilane, gamma-(methacryloyloxy) propyltrimethoxysilane, 3-aminopropyltrimethoxysilane, 4-aminopropylmethyldimethoxysilane or 3-aminopropylmethyldiethoxysilane.

Commercially available examples of hollow ceramic microspheres are Z-Light^{™} Spheres Microspheres commercialized by 3M^{™} (products such as: 3M^{™} Z-Light^{™} Spheres G-3125, G-3150 and G-3500).

According to one embodiment, the blowable crosslinkable silicone composition according to the invention comprises (by weight, relative to the total weight of the composition):
- from 1.99% to 98.99% of a partial mixture of at least one organopolysiloxane **A** having at least two alkenyl groups bonded to silicon per molecule, at least one organosilicon compound **B** having at least two and preferably at least three hydrogen atoms bonded to silicon per molecule, at least one hydrosilylation catalyst **C,** and at least one porogenic agent **D** which is water, a hydrogel, or an aqueous silicone emulsion;
- from 0.01% to 2% of at least one chemical blowing agent **E;** and
- from 1% to 98% of at least one linear polydimethylsiloxane F which has a dynamic viscosity at 25°C of between 50 mPa.s and 100000 mPa.s.

According to another embodiment, the blowable crosslinkable silicone composition according to the invention comprises (by weight, relative to the total weight of the composition):
- from 4.95% to 49.95% of a partial mixture of at least one organopolysiloxane **A** having at least two alkenyl groups bonded to silicon per molecule, at least one organosilicon compound **B** having at least two and preferably at least three hydrogen atoms bonded to silicon per molecule, at least one hydrosilylation catalyst **C,** and at least one porogenic agent **D** which is water, a hydrogel, or an aqueous silicone emulsion;
- from 0.05% to 1.5% of at least one chemical blowing agent **E;** and
- from 50% to 95% of at least one linear polydimethylsiloxane **F** which has a dynamic viscosity at 25°C of between 50 mPa.s and 100000 mPa.s.

According to another embodiment, the blowable crosslinkable silicone composition according to the invention comprises (by weight, relative to the total weight of the composition):
- from 6.9% to 24.9% of a partial mixture of at least one organopolysiloxane **A** having at least two alkenyl groups bonded to silicon per molecule, at least one organosilicon compound **B** having at least two and preferably at least three hydrogen atoms bonded to silicon per molecule, at least one hydrosilylation catalyst **C,** and at least one porogenic agent **D** which is water, a hydrogel, or an aqueous silicone emulsion;
- from 0.1% to 1.0% of at least one chemical blowing agent **E;** and
- from 75% to 93% of at least one linear polydimethylsiloxane **F** which has a dynamic viscosity at 25°C of between 50 mPa.s and 100000 mPa.s.

Within the above mentioned embodiments, the partial mixture of at least one organopolysiloxane **A** having at least two alkenyl groups bonded to silicon per molecule, at least one organosilicon compound **B** having at least two and preferably at least three hydrogen atoms bonded to silicon per molecule, at least one hydrosilylation catalyst **C,** and at least one porogenic agent **D** which is water, a hydrogel, or an aqueous silicone emulsion, can have the following composition (by weight, relative to the total weight of the partial mixture):
- from 40% to 95% of at least one organopolysiloxane A having at least two alkenyl groups bonded to silicon per molecule;
- from 1% to 20% of at least one organosilicon compound **B** having at least two and preferably at least three hydrogen atoms bonded to silicon per molecule;
- from 2 to 400 ppm, of at least one platinum hydrosilylation catalyst **C,** calculated as weight of platinum metal;
- from 0.3% to 2.5% of at least one porogenic agent **D** which is water, a hydrogel, or an aqueous silicone emulsion

More preferentially, said partial mixture can have the following composition (by weight, relative to the total weight of the partial mixture):
- from 50% to 90% of at least one organopolysiloxane **A** having at least two alkenyl groups bonded to silicon per molecule;
- from 3% to 15% of at least one organosilicon compound **B** having at least two and preferably at least three hydrogen atoms bonded to silicon per molecule;
- from 5 ppm and 200 ppm, of at least one platinum hydrosilylation catalyst **C,** calculated as weight of platinum metal;
- from 0.5% to 1.5% of at least one porogenic agent **D** which is water, a hydrogel, or an aqueous silicone emulsion.

The blowable crosslinkable silicone composition according to the invention can be a two-component system, wherein said two-component system is provided in two separate parts P1 and P2 intended to be mixed to form said blowable crosslinkable silicone composition. One of the parts P1 or P2 comprises the at least one hydrosilylation catalyst **C** and does not comprise the at least one organosilicon compound **B.**

Another object of the invention concerns a process for preparing an article made of a silicone foam as disclosed above.

According to a first embodiment, an object of the invention is a process for preparing an article made of a silicone foam, comprising the steps of:
1a) combining the components of the blowable crosslinkable silicone composition according to the invention and as defined above, to provide a precursor of a silicone foam,
1b) fill said precursor of a silicone foam into a mold, and
1c) allowing said precursor of a silicone foam to blow and crosslink.

According to said embodiment, step 1b) can preferably be performed into a mold so that the silicone foam may have the specific desired geometry.

According to a second embodiment, an object of the invention is a process for preparing an article comprising a substrate coated with a silicone foam, comprising the steps of:
2a) combining the components of the blowable crosslinkable silicone composition, according to the invention and as defined above, to provide a precursor of a silicone foam,
2b) coating said precursor of a silicone foam onto the substrate, preferably a woven, nonwoven or composite textile,
2c) allowing said precursor of a silicone foam to blow and crosslink.

Step 1c) and Step 2c) of the process according to the invention (i.e. blowing and crosslinking the precursor into a silicone foam) can be carried out by heating at a temperature range of between 50°C to 200°C, preferably of between 100°C to 170°C. The temperature of step c) can be adapted according to the degradation temperature of the chemical blowing agent **E.** For example, for ammonium hydrogencarbonate, degradation temperature is around 60°C. The temperature of step 1c) and step 2c) can be preferably set above 60°C, so that both blowing and crosslinking can proceed essentially simultaneously. Alternatively, step 1c) and step 2c) can start at room temperature, and the temperature can be raised afterwards so as to control separately blowing by the chemically blowing agent **E** and crosslinking.

Another objective of the invention is to provide a new process for additive manufacturing a 3D-shape article made of said silicone foam. Such process will also enable to manufacture complex shape objects made of such materials.

One object of the present invention relates to a process for additive manufacturing a 3D-shape article made of a silicone foam, comprising the steps of:
3a) printing with a 3D printer a portion of said blowable crosslinkable silicone composition as defined above, to form a deposit into a supporting material **SM** which is a gel or microgel suitable for 3D-gel printing silicone foam, said deposit is achieved by way of a device which has at least one delivery unit which can be positioned in x-, y- and z- directions,
3b) allowing the printed blowable crosslinkable silicone composition to partially or totally blow and crosslink, to obtain a silicone foam deposit within said supporting material **SM,**
3c) optionally repeating several times steps a) and b) until the desired 3D-shape is obtained,
3d) removing mechanically or via dissolution in a solvent said supporting material **SM,** and
3e) recovering a 3D-shape article made of a silicone foam.

According to a specific embodiment, step 3b) can be carried out by heating at a temperature range of between 50°C to 200°C, preferably of between 100°C to 170°C. The temperature of step 3b) can be adapted according to the degradation temperature of the chemical blowing agent **E.** For example, for ammonium hydrogencarbonate, degradation temperature is around 60°C. The temperature of step 3b) can be preferably set above 60°C, so that both blowing and crosslinking can proceed essentially simultaneously. Alternatively, step 3b) can start at room temperature, and the temperature can be raised afterwards so as to control separately blowing by the chemically blowing agent **E** and crosslinking. This specific embodiment could advantageously provide de silicone foam having anisotropic properties.

Printing is preferably carried out layer by layer with a 3D-printer which may be selected from an extrusion 3D printer or a material jetting 3D printer. 3D printing is generally associated with a host of related technologies used to fabricate physical objects from computer generated, e.g. computer- aided design (CAD), data sources. "3D printer" is defined as a machine used for 3D printing, and "3D printing" is defined as the fabrication of objects through the deposition of a material using a print head, nozzle, or another printer technology.

In one preferred embodiment, the method for manufacturing article made of silicone foam according to the invention uses an extrusion 3D printer. The blowable crosslinkable silicone composition is extruded through a nozzle. The nozzle may be heated to aid in dispensing the addition crosslinking silicone composition. The blowable crosslinkable silicone composition to be dispensed through the nozzle may be supplied from a cartridge-like system. It is also possible to use a coaxial cartridges system with a static mixer and only one nozzle. Pressure will be adapted to the fluid to be dispensed, the associated nozzle average diameter and the printing speed. Because of the high shear rate occurring during the nozzle extrusion, the viscosity of the blowable crosslinkable silicone composition is greatly lowered and so permits the printing of fine layers. Cartridge pressure could vary from 1 bar (i.e. atmospheric pressure) to 28 bars, preferably from 1 bar to 10 bars and most preferably from 2 bars to 8 bars. An adapted equipment using aluminum cartridges can be used to resist such a pressure. The nozzle and/or build platform moves in the x-y (horizontal plane) to complete the cross section of the object, before moving in the z- axis (vertical) plane once one layer is complete. The nozzle has a high x-y-z- movement precision around 10 pm. After each layer is printed in the x- and y- work plane, the nozzle is displaced in the z-direction only far enough that the next layer can be applied in the x-, y- work place. In this manner, the 3D article is built one layer at a time from the bottom to the upward. The average diameter of a nozzle is related to the thickness of the layer. In an embodiment, the diameter of the layer is comprised from 50 pm to 2000 pm, preferably from 100 pm to 800 pm and most preferably from 100 pm to 500 pm. Advantageously, printing speed is comprised between 1 mm/s and 50 mm/s, preferably between 5 mm/s and 30 mm/s to obtain the best compromise between good accuracy and manufacture speed.

The supporting material SM is a gel or microgel suitable for 3D-gel printing silicone foam. The gel or microgel provides a constant support for the liquid material during 3D-printing. This allows more complex objects to be printed without the need for added supports, and at a faster pace. The supporting material **SM** may be selected by the person skilled in the art among the materials publicly disclosed, for instance in the international patent applications WO 2019/215190 A1 and WO 2020/127882 A1, or the US patent applications US 2015/0028523 A1, US 2018/0036953 A1, and US 2018/0057682 A1. Further details could also be found in the scientific publication of Arthur Colly, Christophe Marquette, and Edwin-Joffrey Courtial: "Poloxamer/Poly(ethylene glycol) Self-Healing Hydrogel for High-Precision Freeform Reversible Embedding of Suspended Hydrogel" (Langmuir 2021, 37, 14, 4154-4162).

According to one embodiment of the claimed process, the supporting material **SM** may be provided as a matrix, for example into a container, and placed at a required temperature. According to another embodiment, the supporting material **SM** may be delivered simultaneously or at staggered intervals with the blowable crosslinkable silicone composition, at a specific location by way of a device which has at least one delivery unit which can be positioned in x-, y- and z-directions.

The present invention further relates to the use of the silicone foam as defined in the present invention, in the electronics field, in the transportation field, in the aerospace field, in the energy production filed and energy storage field, in the textile and furnishing fields, in the construction field. Examples of uses are the following:
- in the electronic market: potting or encapsulating, sealing, bonding or coating with various kinds of components, as well as high-end precision/sensitive electronic devices such as light-emitting diodes (LED), displays, photovoltaic junction boxes in solar cell modules, diodes, semiconductor devices, relays, sensors, automotive stabilizers, automotive electronic control units (ECUs),

- in the transportation field: automotive, marine or aerospace parts such as hood buffering pads, motor vibration insulators, seats, vibration & noise dampening pieces,
- in the aerospace field: aerospace thermal insulation,
- in the energy production field and energy storage field: high voltage electrical insulation, insulation of secondary battery pack, of stationary energy storage devices, and of charging stations, in solar photovoltaic cells and assemblies, windmills, hydropower assemblies,
- in the textile and furnishing field: insulation-coated and composite textile, artificial leather, footwear and garments insulation, padding,
- in the construction field: construction electrical protection,
- in other fields: cosmetic puffs, medical liquid-absorbing materials, various filters, various sealing elements such as packing, gaskets, o-rings, flexible joints, damping elements, fixing rollers, fixing belts and the like which fix toners on paper by means of heat and/or pressure in image-forming apparatuses of electrophotographic types such as copying machines, printers, facsimile machines.

According to the application, the silicone foam may be used for the manufacture of foamed articles per se, or may be used for the manufacture of composites or laminates with metals, organic resins, or elastic materials. For instance, composite articles can comprise a shell and a filler, wherein said filler consists in or comprises the silicone foam according to the present invention.

Various embodiments of the present invention can be better understood by reference to the following examples which are offered by way of illustration. The present invention is not limited to the examples given herein.

### Examples

### Raw materials:

Organopolysiloxane A1 = mixture of several polydimethylsiloxane oils with dimethylvinylsilyl end-units with viscosity at 25°C varying from about 4,000 mPa to about 100,000 mPa.s, and having an average viscosity at 25°C of about 10,000 mPa.s.
Organopolysiloxane A2 = MD^{Vi}Q branched organopolysiloxane, where the vinyl groups bonded to silicon atoms are carried by the D groups
Organopolysiloxane B1 = poly(methylhydrogen)siloxane with trimethylsilyl end-units with a viscosity at 25°C of about 20 mPa.s
Organopolysiloxane B2 = polydimethylsiloxane with dimethylhydrogensilyl end-units with a viscosity at 25°C of about 7 mPa.s
Catalyst C = 10% by weight of Platinum metal, known as Karstedt's catalyst
Emulsion D = silicone emulsion containing about 59.5wt.% of water
Blowing agent E1 = Ammonium hydrogencarbonate
Blowing agent E2 = Finely grounded ammonium hydrogencarbonate mixed with silica and polydimethylsiloxane oil (NH₄HCO₃ content = 50 wt.%)
Polydimethylsiloxane F1 = PDMS with a viscosity at 25°C of about 1000 mPa.s
Polydimethylsiloxane F2 = PDMS with a viscosity at 25°C of about 5000 mPa.s
Polydimethylsiloxane F3 = mixture of several polydimethylsiloxane oils (PDMS and
polydimethylsiloxanes with one dimethylvinylsilyl end-unit and one trimethylsilyl end-unit) having an average viscosity at 25°C of about 300 mPa.s
Polydimethylsiloxane F4 = mixture of several polydimethylsiloxane oils (PDMS and
polydimethylsiloxanes with one dimethylvinylsilyl end-unit and one trimethylsilyl end-unit) having an average viscosity at 25°C of about 1000 mPa.s
Polydimethylsiloxane F5 = mixture of several polydimethylsiloxane oils (PDMS and
polydimethylsiloxanes with one dimethylvinylsilyl end-unit and one trimethylsilyl end-unit) having an average viscosity at 25°C of about 2000 mPa.s
Polydimethylsiloxane F6 = mixture of several polydimethylsiloxane oils (PDMS and
polydimethylsiloxanes with one dimethylvinylsilyl end-unit and one trimethylsilyl end-unit) having an average viscosity at 25°C of about 20,000 mPa.s

### Comparative example 1:

A blowable crosslinkable silicone composition was prepared by mixing the components as mentioned in Table 1 below:

**Table 1**

| **Comparative Example 1** | |
|---|---|
| Organopolysiloxane A1 | 65.50% |
| Organopolysiloxane A2 | 20.25% |
| Organopolysiloxane B1 | 9.50% |
| Organopolysiloxane B2 | 3.70% |
| Emulsion D | 1.00% |
| Catalyst C | 0.05% |

The obtained hydrogen-blown silicone foam shows a high stiffness.

### Comparative example 2:

The silicone foam disclosed in prior art document WO 2020/072374 in Table 14 was reproduced. The raw materials (linear PDMS, polydimethylsiloxane with dimethylvinylsilyl end-units, platinum catalyst, crosslinker and ammonium hydrogencarbonate) were mixed and temperature was maintained at 150°C for 30 minutes so that the crosslinking and the blowing occurred. The silicone foam is exclusively chemically foamed.

The feel of this material was greatly improved in comparison to the Comparative example 1, but it was found that the material had a very irregular cell structure.

### Examples 1-6:

Blowable crosslinkable silicone compositions were prepared by mixing the components as mentioned in Table 2 below. After mixing, the temperature was maintained at 150°C for 60 minutes.

**Table 2**

| | **Ex. 1** | **Ex. 2** | **Ex.3** | **Ex. 4** | **Ex. 5** | **Ex. 6** |
|---|---|---|---|---|---|---|
| Hydrogen-blown silicone foam composition according to Comparative Example 1 | 10% | 15% | 7% | 10% | 15% | 7% |
| Blowing agent E | 0.125% | 0.125% | 0.125% | 0.25% | 0.25% | 0.25% |
| Polydimethylsiloxane F1 | 90% | 85% | 93% | | | |
| Polydimethylsiloxane F2 | | | | 90% | 85% | 93% |

The silicone foams obtained according to the invention, which are simultaneously hydrogen-blown and chemically blown foams, show low density, low weight, good cell structure and with soft shock-resistant feel.

### Examples 7-9:

Blowable crosslinkable silicone compositions were prepared by mixing the components as mentioned in Table 3 below. After mixing, the temperature was maintained at 150°C for 60 minutes.

**Table 3**

| | **Ex.1 (from Table 2)** | **Ex.7** | **Ex.8** | **Ex.9** |
|---|---|---|---|---|
| Hydrogen-blown silicone foam composition according to Comparative Example 1 | 10% | 10% | 10% | 10% |
| Blowing agent E1 | 0.125% | | | |
| Blowing agent E2 | | 0.25% | 0.25% | 0.25% |
| Polydimethylsiloxane F1 | 90% | 89.75% | 44.875% | |
| Polydimethylsiloxane F3 | | | 44.875% | 89.75% |

### Examples 10-15:

General composition of the hydrogen-blown silicone foam "H2B foam":
- 67.10 wt.% of a mixture of several polydimethylsiloxane oils with dimethylvinylsilyl end-units, having an average viscosity at 25°C of about 1,000 mPa.s;
- 11.2 wt.% of treated fumed silica;
- 0.64 wt.% of water;
- 0.64 wt.% of an emulsifier;
- 0.03 wt.% of Karstedt's catalyst (containing 10% by weight of Platinum metal);
- 20.00 wt.% of poly(methylhydrogen)siloxane with trimethylsilyl end-units with a viscosity at 25°C of about 20 mPa.s;
- 0.39 wt.% of ethynylcyclohexanol.

Blowable crosslinkable silicone compositions were prepared by mixing the components as mentioned in Table 4 below. After mixing, the temperature was maintained at 150°C for 60 minutes.

**Table 4**

| | **Ex.10** | **Ex.11** | **Ex.12** | **Ex.13** | **Ex.14** | **Ex.15** |
|---|---|---|---|---|---|---|
| H2B foam with emulsifier = PVP10 | 6.27% | | | 6.27% | 6.27% | 6.27% |
| H2B foam with emulsifier = PVP40 | | 6.27% | | | | |
| H2B foam with emulsifier = silicone polyether | | | 6.27% | | | |
| Blowing agent E2 | 0.25% | 0.25% | 0.25% | 0.25% | 0.25% | 0.25% |
| Polydimethylsiloxane F3 | 93.48% | 93.48% | 93.48% | | | |
| Polydimethylsiloxane F4 | | | | 93.48% | | |
| Polydimethylsiloxane F5 | | | | | 93.48% | |
| Polydimethylsiloxane F6 | | | | | | 93.48% |

| | | | | | | |
|---|---|---|---|---|---|---|
| PVP10 = polyvinylpyrrolidone; average molecular weight 10,000 PVP40 = polyvinylpyrrolidone; average molecular weight 40,000 | | | | | | |

### Example 16:

Example 10 was reproduced, except that the composition of the hydrogen-blown silicone foam "H2B foam" comprised 14.6 wt.% of treated fumed silica (instead of 11.2 wt.%) and 63.7 wt.% of the same mixture of several polydimethylsiloxane oils (instead of 67.1 wt.%).

The silicone foams obtained with the compositions of Examples 7 to 16 show low density, low weight, and good cell structure.

| | Relative density (at room temperature) |
|---|---|
| Ex.9 | 0.7450 |
| Ex.10 | 0.7489 |
| Ex.11 | 0.7878 |
| Ex.13 | 0.7282 |
| Ex.14 | 0.6070 |
| Ex.15 | 0.6589 |
| Ex.16 | 0.9062 |

## Claims

1. A silicone foam obtained from a blowable crosslinkable silicone composition comprising:
- at least one organopolysiloxane **A** having at least two alkenyl groups bonded to silicon per molecule;
- at least one organosilicon compound **B** having at least two and preferably at least three hydrogen atoms bonded to silicon per molecule;
- at least one hydrosilylation catalyst **C;**
- at least one porogenic agent **D** which is water, a hydrogel, or an aqueous silicone emulsion;
- at least one chemical blowing agent **E;** and
- at least one linear polydimethylsiloxane **F** which has a dynamic viscosity at 25°C of between 50 mPa.s and 100000 mPa.s.

2. The silicone foam according to Claim 1, wherein the organopolysiloxane **A** comprises a linear organopolysiloxane; preferably the organopolysiloxane **A** contains terminal dimethylvinylsilyl units, and more preferably the organopolysiloxane **A** is a poly(dimethylsiloxane) comprising terminal dimethylvinylsilyl groups.

3. The silicone foam according to Claim 1 or Claim 2, wherein the organopolysiloxane **A** comprises a branched organopolysiloxane comprising C₂₋₆ alkenyl units; preferably selected from the group consisting of the silicone resins of following formulas:
- M^{Vi}Q, where the alkenyl groups bonded to silicon atoms are carried by the M groups,
- MM^{Vi}Q, where the alkenyl groups bonded to silicon atoms are carried by a part of the M units,
- MD^{Vi}Q, where the alkenyl groups bonded to silicon atoms are carried by the D groups,
- MDD^{Vi}Q, where the alkenyl groups bonded to silicon atoms are carried by a part of the D groups,
- MM^{Vi}TQ, where the alkenyl groups bonded to silicon atoms are carried by a part of the M units,
- MM^{Vi}DD^{Vi}Q, where the hydrogen atoms bonded to silicon atoms are carried by a part of the M and D units,
- and their mixtures,
with M: R¹₃SiO_{1/2} siloxyl unit, M^{Vi}: siloxyl unit selected from the group consisting of the YR¹₂SiO_{1/2} and Y₂R¹SiO_{1/}, D: R¹₂SiO_{2/2} siloxyl unit, D^{Vi}: siloxyl unit selected from the group consisting of Y₂SiO_{2/2} or YR¹SiO_{2/2} siloxyl units, T: siloxyl unit of formula R¹SiO_{3/2}, and Q: siloxyl unit of formula SiO_{4/2}; the symbol Y is a C₂₋₆ alkenyl, preferably vinyl; and the symbol R¹ is a monovalent hydrocarbon group having from 1 to 12 carbon atoms, preferably selected from the alkyl groups having from 1 to 8 carbon atoms, such as the methyl, ethyl or propyl groups, cycloalkyl groups having from 3 to 8 carbon atoms and aryl groups having from 6 to 12 carbon atoms.

4. The silicone foam according to any one of Claims 1 to 3, wherein the blowable crosslinkable silicone composition comprises a mixture of at least one linear organopolysiloxane having at least two alkenyl groups bonded to silicon per molecule and of at least one branched organopolysiloxane having at least two alkenyl groups bonded to silicon per molecule.

5. The silicone foam according to any one of Claims 1 to 4, wherein the blowable crosslinkable silicone composition comprises a mixture of at least one organosilicon compound **B1** having at least three hydrogen atoms bonded to silicon per molecule and at least one organosilicon compound **B2** having two hydrogen atoms bonded to silicon per molecule.

6. The silicone foam according to any one of Claims 1 to 5, wherein the porogenic agent **D** is an aqueous silicone emulsion.

7. The silicone foam according to any one of Claims 1 to 5, wherein the porogenic agent **D** is a mixture of water and at least one emulsifier

8. The silicone foam according to any one of Claims 1 to 7, wherein the chemical blowing agent **E** is at least one hydrogencarbonate salt, preferably selected from the group consisting of ammonium hydrogencarbonate (NH₄)HCO₃, sodium hydrogencarbonate NaHCO₃, calcium hydrogencarbonate Ca(HCO₃)₂, and mixtures thereof; and more preferably the chemical blowing agent **E** is ammonium hydrogencarbonate.

9. The silicone foam according to any one of Claims 1 to 8, wherein the linear polydimethylsiloxane **F** consists in a mixture of linear polydimethylsiloxanes (I) and (II), with a weight ratio of (I):(II) comprised between 100:0 and 0:100,
wherein the linear polydimethylsiloxane (I) has the following formula:
(CH₃)₃SiO (SiO(CH₃)₂)ₙ Si(CH₃)₃ (I)
in which n is an integer from 50 to 900, and preferably from 50 to 700; and
wherein the linear polydimethylsiloxane (II) has the following formula:
(CH₃)₃SiO (SiO(CH₃)₂)ₙ Si(CH₃)₂(Y) (II)
in which Y is a C₂₋₆ alkenyl, preferably vinyl, and n is an integer from 50 to 900, and preferably from 50 to 700.

10. The silicone foam according to any one of claims 1 to 9, wherein the blowable crosslinkable silicone composition further comprises hollow microspheres, preferably hollow glass microspheres.

11. The silicone foam according to any one of Claims 1 to 10, wherein the blowable crosslinkable silicone composition according to the invention comprises by weight, relative to the total weight of the composition:
- from 1.99% to 98.99% of a partial mixture of at least one organopolysiloxane **A** having at least two alkenyl groups bonded to silicon per molecule, at least one organosilicon compound **B** having at least two and preferably at least three hydrogen atoms bonded to silicon per molecule, at least one hydrosilylation catalyst **C,** and at least one porogenic agent **D** which is water, a hydrogel, or an aqueous silicone emulsion;
- from 0.01% to 2% of at least one chemical blowing agent **E;** and
- from 1% to 98% of at least one linear polydimethylsiloxane **F** which has a dynamic viscosity at 25°C of between 50 mPa.s and 100000 mPa.s.

12. The silicone foam according to Claim 11, wherein the partial mixture of at least one organopolysiloxane **A** having at least two alkenyl groups bonded to silicon per molecule, at least one organosilicon compound **B** having at least two and preferably at least three hydrogen atoms bonded to silicon per molecule, at least one hydrosilylation catalyst **C,** and at least one porogenic agent **D** which is water, a hydrogel, or an aqueous silicone emulsion, has the following composition by weight, relative to the total weight of the partial mixture:
- from 40% to 95% of at least one organopolysiloxane **A** having at least two alkenyl groups bonded to silicon per molecule;
- from 1% to 20% of at least one organosilicon compound **B** having at least two and preferably at least three hydrogen atoms bonded to silicon per molecule;
- from 2 and 400 ppm, of at least one platinum hydrosilylation catalyst **C,** calculated as weight of platinum metal;
- from 0.3% to 2.5% of at least one porogenic agent **D** which is water, a hydrogel, or an aqueous silicone emulsion.

13. A process for preparing an article made of a silicone foam, comprising the steps of:
1a) combining the components of the blowable crosslinkable silicone composition, as defined in any one of Claims 1 to 12, to provide a precursor of a silicone foam,
1b) fill said precursor of a silicone foam into a mold, and
1c) allowing said precursor of a silicone foam to blow and crosslink.

14. A process for preparing an article comprising a substrate coated with a silicone foam, comprising the steps of:
2a) combining the components of the blowable crosslinkable silicone composition, as defined in any one of Claims 1 to 12, to provide a precursor of a silicone foam,
2b) coating said precursor of a silicone foam onto the substrate, preferably a woven, nonwoven or composite textile,
2c) allowing said precursor of a silicone foam to blow and crosslink.

15. A process for additive manufacturing a 3D-shape article made of a silicone foam, comprising the steps of:
3a) printing with a 3D printer a portion of said blowable crosslinkable silicone composition as defined in any one of Claims 1 to 12, to form a deposit into a supporting material **SM** which is a gel or microgel suitable for 3D-gel printing silicone foam, said deposit is achieved by way of a device which has at least one delivery unit which can be positioned in x-, y- and z- directions,
3b) allowing the printed blowable crosslinkable silicone composition to partially or totally blow and crosslink, to obtain a silicone foam deposit within said supporting material **SM,**
3c) optionally repeating several times steps a) and b) until the desired 3D-shape is obtained,
3d) removing mechanically or via dissolution in a solvent said supporting material **SM,** and
3e) recovering a 3D-shape article made of a silicone foam.

16. Use of a silicone foam according to any one of Claims 1 to 12, wherein said silicone foam is used for the manufacture of foamed articles per se, or is used for the manufacture of composites or laminates with metals, organic resins, or elastic materials in the electronics field, in the transportation field, in the aerospace field, in the energy production field, in the energy storage field, in the textile and furnishing fields, and in the construction field.

17. Use according to Claim 12, wherein said use consists in:
- in the electronic market: potting or encapsulating, sealing, bonding or coating with various kinds of components, as well as high-end precision/sensitive electronic devices such as light-emitting diodes (LED), displays, photovoltaic junction boxes in solar cell modules, diodes, semiconductor devices, relays, sensors, automotive stabilizers, automotive electronic control units (ECUs),
- in the transportation field: automotive, marine or aerospace parts such as hood buffering pads, motor vibration insulators, seats, vibration & noise dampening pieces,
- in the aerospace field: aerospace thermal insulation,
- in the energy production field and energy storage field: high voltage electrical insulation, insulation of secondary battery pack, of stationary energy storage devices, and of charging stations, in solar photovoltaic cells and assemblies, windmills, hydropower assemblies,
- in the textile and furnishing field: insulation-coated and composite textile, artificial leather, footwear and garments insulation, padding
- in the construction field: construction electrical protection.

## Patentansprüche

1. Siliconschaum, erhalten aus einer blasbaren, vernetzbaren Siliconzusammensetzung umfassend:
- wenigstens ein Organopolysiloxan A mit wenigstens zwei an Silicium gebundenen Alkenylgruppen pro Molekül;
- wenigstens eine Organosiliciumverbindung B mit wenigstens zwei und vorzugsweise wenigstens drei an Silicium gebundenen Wasserstoffatomen pro Molekül;
- wenigstens einen Hydrosilylierungskatalysator C;
- wenigstens einen Porenbildner D, der Wasser, ein Hydrogel oder eine wässrige Siliconemulsion ist;
- wenigstens ein chemisches Treibmittel E; und
- wenigstens ein lineares Polydimethylsiloxan F mit einer dynamischen Viskosität bei 25 °C von zwischen 50 mPa.s und 100.000 mPa.s.

2. Siliconschaum nach Anspruch 1, wobei das Organopolysiloxan A ein lineares Organopolysiloxan umfasst; vorzugsweise das Organopolysiloxan A terminale Dimethylvinylsilyleinheiten enthält und bevorzugter das Organopolysiloxan A Poly(dimethylsiloxan), das terminale Dimethylvinylsilylgruppen umfasst, ist.

3. Siliconschaum nach Anspruch 1 oder Anspruch 2, wobei das Organopolysiloxan A ein verzweigtes Organopolysiloxan umfasst, das C₂₋₆-Alkenyleinheiten umfasst; vorzugsweise ausgewählt aus der Gruppe bestehend aus den Siliconharzen der folgenden Formeln:
- M^{VI}Q, wobei die an Siliciumatome gebundenen Alkenylgruppen von den M-Gruppen getragen werden;
- MM^{VI}Q, wobei die an Siliciumatome gebundenen Alkenylgruppen von einem Teil der M-Einheiten getragen werden;
- MD^{VI}Q, wobei die an Siliciumatome gebundenen Alkenylgruppen von den D-Gruppen getragen werden;
- MDD^{VI}Q, wobei die an Siliciumatome gebundenen Alkenylgruppen von einem Teil der D-Gruppen getragen werden;
- MM^{VI}TQ, wobei die an Siliciumatome gebundenen Alkenylgruppen von einem Teil der M-Einheiten getragen werden;
- MM^{VI}DD^{VI}Q, wobei die an Siliciumatome gebundenen Wasserstoffatome von einem Teil der M- und D-Einheiten getragen werden;
- und deren Gemischen,
mit M: R¹₃SiO_{1/2}-Siloxyleinheit, M^{VI}: Siloxyleinheit ausgewählt aus der Gruppe bestehend aus YR¹₂SiO_{1/2} und Y₂R¹SiO_{1/}, D: R¹₂SiO_{2/2}-Siloxyleinheit, D^{VI}: Siloxyleinheit ausgewählt aus der Gruppe bestehend aus Y₂SiO_{2/2}- oder YR¹SiO_{2/2}-Siloxyleinheiten, T: Siloxyleinheit der Formel R¹SiO_{3/2} und Q: Siloxyleinheit der Formel SiO_{4/2}; das Symbol Y ist ein C₂₋₆-Alkenyl, vorzugsweise Vinyl; und das Symbol R¹ ist eine einwertige Kohlenwasserstoffgruppe mit 1 bis 12 Kohlenstoffatomen, vorzugsweise ausgewählt aus den Alkylgruppen mit 1 bis 8 Kohlenstoffatomen, wie z.B. die Methyl-, Ethyl- oder Propylgruppen, Cycloalkylgruppen mit 3 bis 8 Kohlenstoffatomen und Arylgruppen mit 6 bis 12 Kohlenstoffatomen.

4. Siliconschaum nach einem der Ansprüche 1 bis 3, wobei die blasbare vernetzbare Siliconzusammensetzung ein Gemisch aus wenigstens einem linearen Organopolysiloxan mit wenigstens zwei an Silicium gebundenen Alkenylgruppen pro Molekül und aus wenigstens einem verzweigten Organopolysiloxan mit wenigstens zwei an Silicium gebundenen Alkenylgruppen pro Molekül umfasst.

5. Siliconschaum nach einem der Ansprüche 1 bis 4, wobei die blasbare vernetzbare Siliconzusammensetzung ein Gemisch aus wenigstens einer Organosiliciumverbindung B1 mit wenigstens drei an Silicium gebundenen Wasserstoffatomen pro Molekül und wenigstens einer Organosiliciumverbindung B2 mit zwei an Silicium gebundenen Wasserstoffatomen pro Molekül umfasst.

6. Siliconschaum nach einem der Ansprüche 1 bis 5, wobei der Porenbildner D eine wässrige Siliconemulsion ist.

7. Siliconschaum nach einem der Ansprüche 1 bis 5, wobei der Porenbildner D ein Gemisch aus Wasser und wenigstens einem Emulgator ist.

8. Siliconschaum nach einem der Ansprüche 1 bis 7, wobei das chemische Treibmittel E wenigstens ein Hydrogencarbonatsalz ist, vorzugsweise ausgewählt aus der Gruppe bestehend aus Ammoniumhydrogencarbonat (NH₄)HCO₃, Natriumhydrogencarbonat NaHCO₃, Calciumhydrogencarbonat Ca(HCO₃)₂ und Gemischen davon; wobei bevorzugter das chemische Treibmittel E Ammoniumhydrogencarbonat ist.

9. Siliconschaum nach einem der Ansprüche 1 bis 8, wobei das lineare Polydimethylsiloxan F aus einem Gemisch von linearen Polydimethylsiloxanen (I) und (II) mit einem Gewichtsverhältnis von (I):(II) in dem Bereich zwischen 100:0 und 0:100 besteht,
wobei das lineare Polydimethylsiloxan (I) die folgende Formel aufweist:
(CH₃)₃SiO (SiO (CH₃)₂)ₙ Si (CH₃)₃ (I)
wobei n eine ganze Zahl von 50 bis 900 und vorzugsweise von 50 bis 700 ist; und
wobei das lineare Polydimethylsiloxan (II) die folgende Formel aufweist:
(CH₃)₃SiO (SiO(CH₃)₂)ₙ Si(CH₃)₂(Y) (II)
wobei Y ein C₂₋₆-Alkenyl, vorzugsweise Vinyl, ist und n eine ganze Zahl von 50 bis 900 und vorzugsweise von 50 bis 700 ist.

10. Siliconschaum nach einem der Ansprüche 1 bis 9, wobei die blasbare vernetzbare Siliconzusammensetzung ferner hohle Mikrokügelchen, vorzugsweise hohle Glasmikrokügelchen, umfasst.

11. Siliconschaum nach einem der Ansprüche 1 bis 10, wobei die blasbare vernetzbare Siliconzusammensetzung gemäß der Erfindung nach Gewicht, bezogen auf das Gesamtgewicht der Zusammensetzung, umfasst:
- von 1,99 % bis 98,99 % an einem Teilgemisch von wenigstens einem Organopolysiloxan A mit wenigstens zwei an Silicium gebundenen Alkenylgruppen pro Molekül, wenigstens einer Organosiliciumverbindung B mit wenigstens zwei und vorzugsweise wenigstens drei an Silicium gebundenen Wasserstoffatomen pro Molekül, wenigstens einem Hydrosilylierungskatalysator C und wenigstens einem Porenbildner D, der Wasser, ein Hydrogel oder eine wässrige Siliconemulsion ist;
- von 0,01 % bis 2 % an wenigstens einem chemischen Treibmittel E; und
- von 1 % bis 98 % an wenigstens einem linearen Polydimethylsiloxan F mit einer dynamischen Viskosität bei 25 °C zwischen 50 mPa.s und 100.000 mPa.s.

12. Siliconschaum nach Anspruch 11, wobei das Teilgemisch von wenigstens einem Organopolysiloxan A mit wenigstens zwei an Silicium gebundenen Alkenylgruppen pro Molekül, wenigstens einer Organosiliciumverbindung B mit wenigstens zwei und vorzugsweise wenigstens drei an Silicium gebundenen Wasserstoffatomen pro Molekül, wenigstens einem Hydrosilylierungskatalysator C und wenigstens einem Porenbildner D, der Wasser, ein Hydrogel, oder eine wässrige Siliconemulsion ist, die folgende Gewichtszusammensetzung bezogen auf das Gesamtgewicht des Teilgemischs aufweist:
- von 40 % bis 95 % an wenigstens einem Organopolysiloxan A mit wenigstens zwei an Silicium gebundenen Alkenylgruppen pro Molekül;
- von 1 % bis 20 % an wenigstens einer Organosiliciumverbindung B mit wenigstens zwei und vorzugsweise wenigstens drei an Silicium gebundenen Wasserstoffatomen pro Molekül;
- von 2 bis 400 ppm an wenigstens einem Platin-Hydrosilylierungskatalysator C, berechnet als Gewicht Platinmetall;
- von 0,3 % bis 2,5 % an wenigstens einem Porenbildner D, der Wasser, ein Hydrogel oder eine wässrige Siliconemulsion ist.

13. Verfahren zur Herstellung eines Gegenstands aus einem Siliconschaum, umfassend die Schritte:
1a) Kombinieren der Komponenten der blasbaren vernetzbaren Siliconzusammensetzung nach einem der Ansprüche 1 bis 12, um einen Vorläufer eines Siliconschaums bereitzustellen,
1b) Füllen des Vorläufers eines Siliconschaums in ein Formwerkzeug, und
1c) Erlauben von Ausblasen und Vernetzen des Vorläufers eines Siliconschaums.

14. Verfahren zur Herstellung eines Gegenstands, der ein mit Siliconschaum beschichtetes Substrat umfasst, umfassend die Schritte:
2a) Kombinieren der Komponenten der blasbaren vernetzbaren Siliconzusammensetzung nach einem der Ansprüche 1 bis 12, um einen Vorläufer eines Siliconschaums bereitzustellen,
2b) Schichten des Vorläufers eines Siliconschaums auf das Substrat, vorzugsweise ein Gewebe-, Vlies- oder Verbundtextil,
2c) Erlauben von Ausblasen und Vernetzen des Vorläufers eines Siliconschaums.

15. Verfahren zur additiven Fertigung eines Gegenstands mit 3D-Form aus einem Siliconschaum, umfassend die Schritte:
3a) mit einem 3D-Drucker Drucken eines Teils der blasbaren vernetzbaren Siliconzusammensetzung nach einem der Ansprüche 1 bis 12, um eine Abscheidung zu bilden, in ein Trägermaterial SM, das ein Gel oder Mikrogel ist, das zum 3D-Geldruck von Siliconschaum geeignet ist, wobei die Abscheidung mithilfe einer Vorrichtung gebildet wird, die wenigstens eine Abgabeeinheit aufweist, die in x-, y- und z-Richtung positionierbar ist,
3b) Erlauben, dass die gedruckte blasbare vernetzbare Siliconzusammensetzung teilweise oder vollständig ausbläst und vernetzt, um eine Siliconschaumabscheidung innerhalb des Trägermaterials SM zu erhalten,
3c) gegebenenfalls mehrmals Wiederholen der Schritte a) und b), bis die gewünschte 3D-Form erhalten ist,
3d) mechanisch oder durch Auflösen in einem Lösungsmittel Entfernen des Trägermaterials SM, und
3e) Gewinnen eines Gegenstands mit 3D-Form aus einem Siliconschaum.

16. Verwendung eines Siliconschaums nach einem der Ansprüche 1 bis 12, wobei der Siliconschaum zur Herstellung von geschäumten Gegenständen an sich verwendet wird oder zur Herstellung von Verbundstoffen oder Laminaten mit Metallen, organischen Harzen oder elastischen Materialien im Elektronikbereich, im Transportbereich, im Luft- und Raumfahrtbereich, im Energieerzeugungsbereich, im Energiespeicherbereich, im Textil- und Einrichtungsbereich und im Baubereich verwendet wird.

17. Verwendung nach Anspruch 12, wobei die Verwendung besteht aus:
- auf dem Elektronikmarkt: Vergießen oder Verkapseln, Versiegeln, Kleben oder Beschichten mit verschiedenen Arten von Komponenten, sowie hochwertigen Präzisions-/empfindlichen Elektronikvorrichtungen, wie z.B. Leuchtdioden (LED), Anzeigen, Photovoltaik-Verbindungsboxen in Solarzellen-Modulen, Dioden, Halbleitervorrichtungen, Relais, Sensoren, Automobilstabilisatoren, elektronische Steuereinheiten (ECUs) für Automobile,
- im Transportbereich: Teile für Automobile, die Schifffahrt oder die Luft- und Raumfahrt, wie z.B. Pufferpolster für Motorhauben, Motorvibrationsisolatoren, Sitze, vibrations- und schalldämpfende Teile,
- im Luft- und Raumfahrtbereich: Wärmedämmung für die Luft- und Raumfahrt,
- im Energieerzeugungsbereich und Energiespeicherbereich: elektrische Hochspannungsisolierung, Isolierung von Sekundärbatterien, von stationären Energiespeichern und von Ladestationen, in Solarzellen und -Baugruppen, Windkraftanlagen und Wasserkraftanlagen;
- im Textil- und Möbelbereich: isolierbeschichtete und zusammengesetzte Textilien, Kunstleder, Isolierung von Schuhen und Kleidungsstücken, Polsterung
- im Baubereich: elektrischer Schutz im Bauwesen.

## Revendications

1. Mousse de silicone obtenue à partir d'une composition de silicone gonflable réticulable comprenant :
- au moins un organopolysiloxane **A** ayant au moins deux groupes alcényle liés au silicium par molécule ;
- au moins un composé organosilicium **B** ayant au moins deux et de préférence au moins trois atomes d'hydrogène liés au silicium par molécule ;
- au moins un catalyseur d'hydrosilylation **C** ;
- au moins un agent porogène **D** qui est l'eau, un hydrogel ou une émulsion aqueuse de silicone ;
- au moins un agent gonflant chimique **E** ; et
- au moins un polydiméthylsiloxane linéaire F ayant une viscosité dynamique à 25 **°C** comprise entre 50 mPa.s et 100 000 mPa.s.

2. Mousse de silicone selon la revendication 1, dans laquelle l'organopolysiloxane **A** comprend un organopolysiloxane linéaire ; de préférence l'organopolysiloxane **A** contient des motifs diméthylvinylsilyle terminaux, et plus préférablement l'organopolysiloxane **A** est un poly(diméthylsiloxane) comprenant des groupes diméthylvinylsilyle terminaux.

3. Mousse de silicone selon la revendication 1 ou la revendication 2, dans laquelle l'organopolysiloxane **A** comprend un organopolysiloxane ramifié comprenant des motifs alcényle en C₂₋₆ ; de préférence choisi dans le groupe constitué par les résines de silicone de formules suivantes :
- M^{Vi}Q, où les groupes alcényle liés aux atomes de silicium sont portés par les groupes M,
- MM^{Vi}Q, où les groupes alcényle liés aux atomes de silicium sont portés par une partie des motifs M,
- MD^{Vi}Q, où les groupes alcényle liés aux atomes de silicium sont portés par les groupes D,
- MDD^{Vi}Q, où les groupes alcényle liés aux atomes de silicium sont portés par une partie des groupes D,
- MM^{Vi}TQ, où les groupes alcényle liés aux atomes de silicium sont portés par une partie des motifs M,
- MM^{Vi}DD^{Vi}Q, où les atomes d'hydrogène liés aux atomes de silicium sont portés par une partie des motifs M et D,
- et leurs mélanges,
avec M : motif siloxyle R¹₃SiO_{1/2}, M^{Vi} : motif siloxyle choisi dans le groupe constitué par YR¹₂SiO_{1/2} et Y₂R¹SiO_{1/}, D : motif siloxyle R¹₂SiO_{2/2}, D^{Vi} : motif siloxyle choisi dans le groupe constitué par les motifs siloxyle Y₂SiO_{2/2} ou YR¹SiO_{2/2}, T : motif siloxyle de formule R¹SiO_{3/2}, et Q : motif siloxyle de formule SiO_{4/2} ; le symbole Y est un alcényle en C₂₋₆, de préférence vinyle ; et le symbole R¹ est un groupe hydrocarboné monovalent ayant de 1 à 12 atomes de carbone, de préférence choisi parmi les groupes alkyle ayant de 1 à 8 atomes de carbone, tels que les groupes méthyle, éthyle ou propyle, les groupes cycloalkyle ayant de 3 à 8 atomes de carbone et les groupes aryle ayant de 6 à 12 atomes de carbone.

4. Mousse de silicone selon l'une quelconque des revendications 1 à 3, dans laquelle la composition de silicone réticulable gonflable comprend un mélange d'au moins un organopolysiloxane linéaire ayant au moins deux groupes alcényle liés au silicium par molécule et d'au moins un organopolysiloxane ramifié ayant au moins deux groupes alcényle liés au silicium par molécule.

5. Mousse de silicone selon l'une quelconque des revendications 1 à 4, dans laquelle la composition de silicone réticulable gonflable comprend un mélange d'au moins un composé organosilicium **B1** ayant au moins trois atomes d'hydrogène liés au silicium par molécule et d'au moins un composé organosilicium **B2** ayant deux atomes d'hydrogène liés au silicium par molécule.

6. Mousse de silicone selon l'une quelconque des revendications 1 à 5, dans laquelle l'agent porogène **D** est une émulsion aqueuse de silicone.

7. Mousse de silicone selon l'une quelconque des revendications 1 à 5, dans laquelle l'agent porogène **D** est un mélange d'eau et d'au moins un émulsifiant.

8. Mousse de silicone selon l'une quelconque des revendications 1 à 7, dans laquelle l'agent gonflant chimique **E** est au moins un sel d'hydrogénocarbonate, de préférence choisi dans le groupe constitué par l'hydrogénocarbonate d'ammonium (NH₄)HCO₃, l'hydrogénocarbonate de sodium NaHCO₃, l'hydrogénocarbonate de calcium Ca(HCO₃)₂, et des mélanges correspondants ; et plus préférablement l'agent gonflant chimique **E** est l'hydrogénocarbonate d'ammonium.

9. Mousse de silicone selon l'une quelconque des revendications 1 à 8, dans laquelle le polydiméthylsiloxane linéaire **F** consiste en un mélange de polydiméthylsiloxanes linéaires (I) et (II), en un rapport pondéral de (I):(II) compris entre 100:0 et 0:100,
dans laquelle le polydiméthylsiloxane linéaire (I) a la formule suivante :
(CH₃)₃SiO (SiO(CH₃)₂)ₙ Si(CH₃)₃ (I)
dans laquelle n est un entier de 50 à 900, et de préférence de 50 à 700 ; et
dans laquelle le polydiméthylsiloxane linéaire (II) a la formule suivante :
(CH₃)₃SiO (SiO(CH₃)₂)ₙ Si(CH₃)₂(Y) (II)
dans laquelle Y est un alcényle en C₂₋₆, de préférence vinyle, et n est un entier de 50 à 900, et de préférence de 50 à 700.

10. Mousse de silicone selon l'une quelconque des revendications 1 à 9, dans laquelle la composition de silicone réticulable gonflable comprend en outre des microsphères creuses, de préférence des microsphères de verre creuses.

11. Mousse de silicone selon l'une quelconque des revendications 1 à 10, dans laquelle la composition de silicone réticulable gonflable selon l'invention comprend en poids, par rapport au poids total de la composition :
- de 1,99 % à 98,99 % d'un mélange partiel d'au moins un organopolysiloxane **A** ayant au moins deux groupes alcényle liés au silicium par molécule, d'au moins un composé organosilicium **B** ayant au moins deux et de préférence au moins trois atomes d'hydrogène liés au silicium par molécule, d'au moins un catalyseur d'hydrosilylation **C,** et d'au moins un agent porogène **D** qui est l'eau, un hydrogel ou une émulsion aqueuse de silicone ;
- de 0,01 % à 2 % d'au moins un agent gonflant chimique **E** ; et
- de 1 % à 98 % d'au moins un polydiméthylsiloxane linéaire **F** qui a une viscosité dynamique à 25 °C comprise entre 50 mPa.s et 100 000 mPa.s.

12. Mousse de silicone selon la revendication 11, dans laquelle le mélange partiel d'au moins un organopolysiloxane **A** ayant au moins deux groupes alcényle liés au silicium par molécule, d'au moins un composé organosilicium **B** ayant au moins deux et de préférence au moins trois atomes d'hydrogène liés au silicium par molécule, d'au moins un catalyseur d'hydrosilylation **C,** et d'au moins un agent porogène **D** qui est l'eau, un hydrogel, ou une émulsion aqueuse de silicone, a la composition pondérale suivante, par rapport au poids total du mélange partiel :
- de 40 % à 95 % d'au moins un organopolysiloxane **A** ayant au moins deux groupes alcényle liés au silicium par molécule ;
- de 1 % à 20 % d'au moins un composé organosilicium **B** ayant au moins deux et de préférence au moins trois atomes d'hydrogène liés au silicium par molécule ;
- de 2 à 400 ppm, d'au moins un catalyseur d'hydrosilylation au platine **C,** calculé en poids de platine métal ;
- de 0,3 % à 2,5 % d'au moins un agent porogène **D** qui est l'eau, un hydrogel ou une émulsion aqueuse de silicone.

13. Procédé de préparation d'un article fait d'une mousse de silicone, comprenant les étapes de :
1a) combinaison des composants de la composition de silicone réticulable gonflable, telle que définie dans l'une quelconque des revendications 1 à 12, pour fournir un précurseur d'une mousse de silicone,
1b) remplissage dudit précurseur d'une mousse de silicone dans un moule, et
1c) le fait de permettre audit précurseur d'une mousse de silicone de gonfler et réticuler.

14. Procédé de préparation d'un article comprenant un substrat revêtu par une mousse de silicone, comprenant les étapes de :
2a) combinaison des composants de la composition de silicone réticulable gonflable, telle que définie dans l'une quelconque des revendications 1 à 12, pour fournir un précurseur d'une mousse de silicone,
2b) revêtement dudit précurseur d'une mousse silicone sur le substrat, de préférence un textile tissé, non tissé ou composite,
2c) le fait de permettre audit précurseur d'une mousse de silicone de gonfler et réticuler.

15. Procédé de fabrication additive d'un article de forme en 3D composé d'une mousse de silicone, comprenant les étapes de :
3a) impression avec une imprimante 3D d'une partie de ladite composition de silicone réticulable gonflable telle que définie dans l'une quelconque des revendications 1 à 12, pour former un dépôt dans un matériau support **SM** qui est un gel ou un microgel approprié pour l'impression de gel en 3D d'une mousse de silicone, ledit dépôt étant réalisé au moyen d'un dispositif qui possède au moins une unité de délivrance qui peut être positionnée dans les directions x, y et z,
3b) le fait de permettre à la composition silicone réticulable gonflable imprimée de gonfler et réticuler partiellement ou totalement, pour obtenir un dépôt de mousse de silicone dans ledit matériau support **SM,**
3c) éventuellement répétition plusieurs fois des étapes a) et b) jusqu'à obtention de la forme en 3D souhaitée,
3d) élimination mécaniquement ou par dissolution dans un solvant dudit matériau de support **SM,** et
3e) récupération d'un article de forme en 3D fait de mousse de silicone.

16. Utilisation d'une mousse de silicone selon l'une quelconque des revendications 1 à 12, dans laquelle ladite mousse de silicone est utilisée pour la fabrication d'articles en mousse à proprement parler, ou pour la fabrication de composites ou stratifiés avec des métaux, des résines organiques ou des matériaux élastiques dans le domaine de l'électronique, dans le domaine des transports, dans le domaine de l'aérospatiale, dans le domaine de la production d'énergie, dans le domaine du stockage d'énergie, dans le domaine du textile et de l'ameublement, et dans le domaine de la construction.

17. Utilisation selon la revendication 12, dans laquelle ladite utilisation consiste en :
- dans le marché de l'électronique : enrobage ou encapsulation, scellement, liaison ou revêtement avec divers types de composants, ainsi que des dispositifs électroniques de précision/sensibles haut de gamme tels que des diodes émettrices de lumière (LED), des affichages, des boîtes de jonction photovoltaïque dans des modules de cellules solaires, des diodes, des dispositifs semi-conducteurs, des relais, des capteurs, des stabilisateurs d'automobile, des unités de commande électronique d'automobile (ECU)
- dans le domaine des transports : pièces automobiles, marines ou aérospatiales telles que des tampons de capot, des isolateurs de vibrations de moteur, des sièges, des pièces d'amortissement de vibrations et de bruit,
- dans le domaine de l'aérospatial : isolation thermique aérospatiale,
- dans le domaine de la production d'énergie et du stockage d'énergie : isolation électrique à haute tension, isolation de batteries secondaires, de dispositifs fixes de stockage d'énergie et de stations de recharge, dans des cellules et ensembles photovoltaïques solaires, des éoliennes, des ensembles hydroélectriques,
- dans le domaine du textile et de l'ameublement : textile à revêtement isolant et composite, cuir artificiel, isolation de chaussures et de vêtements, rembourrage,
- dans le domaine de la construction : protection électrique de construction.
